# EUROPÄISCHE PATENTSCHRIFT

(11) **EP 1 747 188 B1**
(45) Veröffentlichungstag und Bekanntmachung des Hinweises auf die Patenterteilung: **08.07.2015**
(21) Anmeldenummer: 05744816.9
(22) Anmeldetag: 28.04.2005
(51) Int. Cl.: C07C 209/00, C07C 209/68

(54) **VERFAHREN ZUR HERSTELLUNG VON LANGKETTIGEN QUATERNÄREN AMMONIUM-OXALATEN UND -HYDROGENOXALATEN**
METHOD FOR THE PRODUCTION OF LONG-CHAINED QUATERNARY AMMONIUM OXALATES AND AMMONIUM HYDROGENOXALATES
PROCEDE DE PRODUCTION D'OXALATES D'AMMONIUM ET D'HYDROGENO-OXALATES D'AMMONIUM QUATERNAIRES A LONGUES CHAINES

(30) Priorität: 12.05.2004 DE 102004023417
(43) Veröffentlichungstag der Anmeldung: 31.01.2007
(73) Patentinhaber: Clariant Produkte (Deutschland) GmbH, 65929 Frankfurt am Main (DE)
(72) Erfinder: RAAB, Klaus, 84508 Burgkirchen (DE)
(74) Vertreter: Mikulecky, Klaus
(86) Internationale Anmeldenummer: PCT/EP2005/004535
(87) Internationale Veröffentlichungsnummer: WO 2005/113480

(56) Entgegenhaltungen:
- EP-A- 0 345 475
- US-A1- 2003 165 943

## Beschreibung

Die vorliegende Erfindung betrifft ein neues Verfahren zur Herstellung von langkettigen quaternären Ammoniumoxalaten und von langkettigen quaternären Ammoniumhydrogenoxalaten durch Umsetzung von Aminen mit Dimethylcarbonat und weitere Umsetzung des Zwischenprodukts mit Oxalsäure oder Oxalsäuredihydrat.

JP-A-2002 179 614 offenbart ein Verfahren zur Herstellung von quaternären Ammoniumsalzen mit Carboxylaten als Gegenionen aus quaternären Ammoniumhalogeniden und den Alkalimetallsalzen von C₁- bis C₆-Carbonsäuren in Wasser/Alkohol-Mischungen mit nachträglicher Extraktion. Dabei lässt sich ein Halogenidrestgehalt im Endprodukt nicht vermeiden.

Quaternäre Ammoniumoxalate der Formel

[NR₁R₂R₃R]₂^{⊕} C₂O₄^{2⊖}

oder quaternäre Ammoniumhydrogenoxalate der Formel

[NR₁R₂R₃R_{4]}^{⊕} HC₂O₄^{⊖}

worin R₁, R₂, R₃, R₄ für Alkylgruppen stehen können aus Aminen und einem Alkylierungsmittel wie beispielsweise Alkylchlorid, Alkylbromid oder Alkyliodid und nachträglichem Austausch der Anionen Chlorid, Bromid oder Iodid gegen Oxalat oder Hydrogenoxalat beispielsweise mit Hilfe einer mit Oxalat oder Hydrogenoxalat beladenen Anionenaustauschersäule hergestellt werden. Da es sich bei diesem Anionenaustausch um einen Gleichgewichtsprozess handelt, ist ein vollständiger Austausch der Anionen Chlorid, Bromid oder Iodid gegen Oxalat oder Hydrogenoxalat nur sehr schwierig zu erreichen.

NR₁R₂R₃ + R₄^{-Hal} → [NR₁R₂R₃R₄]^{⊕} Hal^{⊖} Hal^{⊖} =Cl^{⊖}, Br^{⊖}, I^{⊖}

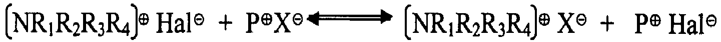

P^{⊕} = polymerer Anionenaustauscher
X^{⊖} = ½ C₂O₄^{2⊖} oder HC₂O₄^{⊖}

Der Anionenaustauscher muss nach dem Ionenaustauschprozess wieder mit Oxalatlösung regeneriert werden. Da quaternäre Ammoniumverbindungen mit langen Alkylgruppen am Stickstoffatom auf diversen Oberflächen, auch auf der großen inneren Oberfläche von Ionenaustauscherharzen, gut adsorbieren, sind außerdem große Mengen an Solvens erforderlich, um die quaternären Ammoniumoxalate oder -hydrogenoxalate vollständig zu eluieren.

Eine weitere Synthesemöglichkeit für quaternäre Ammoniumoxalate und -hydrogenoxalate stellt die Neutralisation von quaternären Ammoniumhydroxiden mit der entsprechenden Menge Oxalsäure H₂C₂O₄ dar.

2 [NR₁R₂R₃R₄]^{⊕}OH^{⊖} + H₂C₂O₄ → [NR₁R₂R₃R₄]₂^{⊕} C₂O₄^{2⊖} + 2 H₂O

[NR₁R₂R₃R₄]eOH^{⊖} + H₂C₂O₄ → [NR₁R₂R₃R₄]^{⊕} HC₂O₄^{⊖} + H₂O

Die quaternären Ammoniumhydroxide können aus quaternären Ammoniumhalogeniden durch Anionenaustausch beispielsweise mit Hilfe einer mit Hydroxid beladenen Anionenaustauschersäule hergestellt werden. 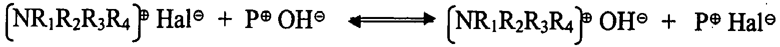
P^{⊕} = polymerer Anionenaustauscher
Hal^{⊖} = Cl^{⊖}, Br^{⊖}, I^{⊖}

Die oben beschriebenen Nachteile wie unvollständiger Anionenaustausch, Adsorption der langkettigen quaternären Ammoniumsalze und großer Solvensverbrauch treten auch hier auf.

EP 0 251 577 B1 beansprucht quaternäre Ammoniumsalze mit speziellen Anionen als Elektrolyte für einen Aluminium-Elektrolytkondensator. Deren Synthese erfolgt durch Neutralisation von kurzkettigen quaternären Ammoniumhydroxiden mit den entsprechenden Säuren wie z.B. Borsäure, Phosphonsäuren, Kieselsäure.

Ein anderer in der Literatur beschriebener Syntheseweg für quaternäre Ammoniumverbindungen ist die Umsetzung von Aminen mit Dialkylcarbonaten zu quaternären Ammoniumalkylcarbonaten und deren weitere Umsetzung mit Säuren HY.

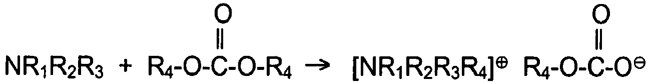

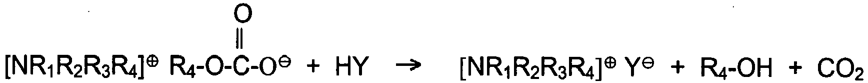

Die deutsche Patentanmeldung B 24673 IV c/12 q (1953) beschreibt die Reaktion von tertiären Aminen mit Estern der Kohlensäure wie beispielsweise Dimethylcarbonat, die Isolierung der quaternären Ammoniumsalze der Kohlensäure und deren weitere Umsetzung mit Säuren wie z.B. Weinsäure.

IT 1153530 offenbart die Herstellung von quaternären Ammoniumalkylcarbonaten aus Aminen und Dialkylcarbonaten und deren weitere Reaktion mit organischen oder anorganischen Säuren.

In der Patentanmeldung EP 0 291 074 A2 ist die Umsetzung von tertiären Aminen oder Phosphinen mit Dialkylcarbonaten zu quaternären Alkylcarbonaten beschrieben, die dann mit einer großen Zahl an verschiedenen anorganischen oder organischen Säuren zu verschiedenen quaternären Ammonium- oder quaternären Phosphoniumsalzen umgesetzt werden.

EP 0 227 179 B1 offenbart die Verwendung von quaternären Ammoniumalkylcarbonaten oder quaternären Ammoniumbenzylcarbonaten als Korrosionsinhibitoren sowie deren Herstellung durch Umsetzung von sekundären oder tertiären Aminen mit Dialkylcarbonaten oder Dibenzylcarbonat.

EP-A-0 345 475 offenbart ein Verfahren zur Herstellung von quaternären Ammoniumsalzen vom Typ [N(CH₃)R₁R₂R₃]^{⊕} RCOO^{⊖}, wobei R ein aliphatischer Kohlenwasserstoffrest mit 8 bis 40 C-Atomen ist, aus tertiären Aminen und Dimethyl- oder Methylethylcarbonat und weitere Umsetzung mit einer langkettigen aliphatischen Carbonsäure RCOOH.

WO 02/00599 A1 und WO 02/00600 A1 offenbaren quaternäre Ammonium- und quaternäre Phosphoniumsalze als wesentliche Bestandteile einer neuen Formulierung sowie deren Verwendung als Mittel zur Stabilisierung und Isolierung von Nukleinsäuren aus Mikroorganismen. Als Anionen dieser quaternären Ammoniumsalze werden Bromid, Chlorid, Phosphat, Sulfat, Formiat, Acetat, Propionat, Oxalat, Malonat, Succinat oder Citrat bevorzugt. Die Herstellung der quaternären Ammoniumoxalate wird nicht beschrieben.

Wegen der beschriebenen Nachteile der Anionaustauschverfahren wie beispielsweise beim Einsatz von Anionenaustauschern, bestand die vorliegender Erfindung zugrunde liegende Aufgabe darin, ein verbessertes Herstellverfahren für langkettige quaternäre Ammoniumoxalate und von langkettigen quaternären Ammoniumhydrogenoxalaten zu entwickeln.

Überraschenderweise wurde gefunden, dass diese Aufgabe gelöst werden kann mit einem Verfahren, bei dem Amine mit Dimethylcarbonat umgesetzt und das Zwischenprodukt, das isoliert werden kann, aber nicht isoliert werden muss, mit Oxalsäure oder Oxalsäuredihydrat weiter zu quaternären Ammoniumoxalaten oder quaternären Ammoniumhydrogenoxalaten umgesetzt werden.

Gegenstand der Erfindung ist daher ein Verfahren zur Herstellung von quaternären Ammoniumoxalaten oder von quaternären Ammoniumhydrogenoxalaten der Formel (1) 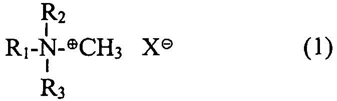 durch Umsetzung von Aminen der Formel (2) 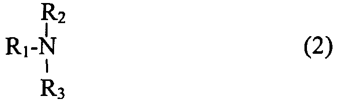 mit Dimethylcarbonat
und weitere Umsetzung des Zwischenprodukts mit Oxalsäure oder Oxalsäuredihydrat worin
X^{⊖} ½ C₂O₄^{2⊖} oder HC₂O₄^{⊖},
- R₁: einen geradkettigen oder verzweigten, aliphatischen Kohlenwasserstoffrest mit 8 bis 22 C-Atomen, der gesättigt ist oder eine, zwei oder drei C=C-Doppelbindungen enthalten kann,
- R₂: CH₃, C₂H₅, C₃H₇, C₄H₉ oder die für R₁ angegebene Bedeutung, und
- R₃: CH₃, C₂H₅, C₃H₇, C₄H₉
bedeuten, wobei die Umsetzung des Amins der Formel (2) mit Dimethylcarbonat unter dem sich dabei im geschlossenen Reaktionsgefäß einstellenden Druck und die weitere Umsetzung des isolierten oder nicht isolierten Zwischenprodukts mit Oxalsäure oder Oxalsäuredihydrat in einem Solvens durchgeführt wird.

R₁ und R₂ stehen vorzugsweise für einen Alkylrest der Formel CₙH₂ₙ₊₁ mit n = 8 bis 14, wobei R₁ und R₂ gleich oder verschieden sein können und R₃ für CH₃ oder H steht. Besonders bevorzugt steht R₁ für einen Alkylrest der Formel CₙH₂ₙ₊₁ mit n = 8 bis 22, wobei dann R₂ und R₃ beide CH₃ bedeuten. Ganz besonders bevorzugt steht R₁ für C₁₄H₂₉ und R₂ und R₃ beide für CH₃.

Die Umsetzung des Amins der Formel (2) mit Dimethylcarbonat kann ohne Zusatz eines weiteren Solvens oder in überschüssigem Dimethylcarbonat als Solvens oder in einem Alkohol als Solvens, bevorzugt in einem kurzkettigen Alkohol mit 1 bis 4 C-Atomen oder besonders bevorzugt in Methanol als Solvens durchgeführt werden. Das Massenverhältnis von Solvens zur Summe der Massen von Amin der Formel (2) und Dimethylcarbonat kann zweckmäßigerweise zwischen 0:1 bis 3:1 liegen und im Falle von Methanol als Solvens bevorzugt zwischen 0:1 bis 2:1, besonders bevorzugt zwischen 0,2:1 bis 1:1. Dimethylcarbonat wirkt in der Regel nur bei erhöhten Temperaturen als Solvens. Bei der Umsetzung von tertiären Aminen der Formel (2) mit R₃ liegt das Molverhältnis von tertiärem Amin zu Dimethylcarbonat bevorzugt zwischen 1:1 und 1:2, besonders bevorzugt zwischen 1:1,2 bis 1:1,7. Im Falle von sekundären Aminen der Formel (2) mit R₃=H (nicht erfindungsgemäß) liegt das

Molverhältnis von sekundärem Amin zu Dimethylcarbonat bevorzugt zwischen 1:2 bis 1:4, besonders bevorzugt zwischen 1:2,5 bis 1:3,5.

Die Reaktionstemperatur der Umsetzung von Aminen der Formel (2) mit Dimethylcarbonat liegt im Allgemeinen zwischen 100 und 180°C, bevorzugt zwischen 120 und 160°C. Der Reaktorinhalt wird unter dem sich einstellenden Eigendruck gut durchmischt, beispielsweise durch Rühren. Die erforderliche Reaktionszeit kann durch analytische Bestimmung des noch unumgesetzten Amins, beispielsweise durch Titration, ermittelt werden.

Das Zwischenprodukt aus der Umsetzung von Aminen der Formel (2) mit Dimethylcarbonat kann auch ohne Isolierung, ohne weitere Reinigung oder ohne Abtrennung vom Solvens mit Oxalsäure oder Oxalsäuredihydrat umgesetzt werden. Dabei kann feste Oxalsäure, festes Oxalsäuredihydrat oder in einem Solvens gelöste Oxalsäure oder Oxalsäuredihydrat zu einer Lösung des Zwischenprodukts in Alkohol, bevorzugt in Methanol, unter Mischen dosiert werden. Bevorzugtes Solvens für Oxalsäure oder Oxalsäuredihydrat ist Wasser. Es ist auch möglich, Oxalsäure oder Oxalsäuredihydrat, beispielsweise gelöst in Wasser, vorzulegen und das Zwischenprodukt, noch heiß in Dimethylcarbonat oder auf Raumtemperatur abgekühlt und gelöst in Alkohol, zuzugeben. Das Molverhältnis von Amin der Formel (2) zu Oxalsäure oder Oxalsäuredihydrat wird je nach Zielprodukt oder gewünschtem Verhältnis von Oxalat zu Hydrogenoxalat in der Regel zwischen 0,9:1 und 2,1:1 gewählt werden. Zur Herstellung von langkettigen quaternären Ammoniumoxalaten der Formel (1) X^{⊖} =½ C₂O₄^{2⊖} wird wird das Molverhältnis von Amin der Formel (2) zu Oxalsäure oder Oxalsäuredihydrat zwischen 1,8:1 und 2,1:1 und zur Herstellung von langkettigen quaternären Ammoniumhydrogenoxalaten der Formel (1) mit X^{⊖} = HC₂O₄^{⊖} zwischen 0,9:1 und 1,1:1 liegen.

Die Reaktionstemperatur zur Umsetzung des Zwischenprodukts wählt man vorteilhaft zwischen 10 bis 80°C, bevorzugt zwischen 20 bis 60°C. Um die Schaumbildung möglichst zu unterdrücken, sollte die Durchmischung nicht zu kräftig sein und die kontinuierliche Dosierung einer oder beider Reaktionskomponenten nicht zu rasch erfolgen. Der Reaktionsdruck ist nicht kritisch. Die Reaktion kann zweckmäßigerweise bei Normaldruck unter Ableitung von Reaktionsgasen erfolgen.

Falls es wünschenswert ist, Methanol, überschüssiges Dimethylcarbonat und die verwendeten Solventien wie die Alkohole oder einen Teil des Wassers von den erfindungsgemäßen Verbindungen der Formel (1) abzutrennen, so kann das durch Destillation erfolgen. Die Destillation kann im Vakuum, bei Normaldruck oder unter Druck erfolgen. Abhängig von den angestrebten Konzentrationen der erfindungsgemäßen Verbindungen der Formel (1), deren Phasenverhalten und von den angestrebten, möglicherweise sehr niedrigen Konzentrationen an Methanol, Dimethylcarbonat oder Alkoholen im Endprodukt, kann es von Vorteil sein, Wasser kontinuierlich oder diskontinuierlich dem Destillationssumpf zuzusetzen. Da die erfindungsgemäßen Verbindungen der Formel (1) in der Regel in Wasser schäumen und abhängig von der Konzentration und vom Solvens Gelphasen bilden können, ist bei einer Destillation der Einsatz eines einstufigen oder mehrstufigen Dünnschichtverdampfers bevorzugt, wobei besonders bevorzugt bei Normaldruck oder im Vakuum destilliert wird. Ein weiterer Vorteil des Dünnschichtverdampfers ist die nur kurzzeitige thermische Belastung der Produkte.

Die Erfindung wird nun an Beispielen noch näher erläutert.

### Beispiel 1:

Eine klare Lösung aus 983 g (4,0 mol) Tetradecyldimethylamin, 540 g (6,0 mol) Dimethylcarbonat und 659 g Methanol wurde bei Raumtemperatur in einen gereinigten, wasserfreien 4,5 Liter-Edelstahl-Schüttelautoklaven eingefüllt. Nach einer Dichtigkeitsprüfung wurde unter einer N₂-Schutzgasatmosphäre und unter Schütteln langsam auf 140°C Innentemperatur aufgeheizt. Der Autoklaveninhalt wurde 4 Stunden lang bei 140°C geschüttelt. Der Innendruck stieg bis auf ca. 20 bar an. Nach dem Abkühlen auf Raumtemperatur wurde der Autoklav vorsichtig entspannt und entleert. Auswaage: 2122 g.

Ein Teil (2000 g) des blassgelben, klaren Autoklaveninhalts wurde in einem 6 Liter-Glasrundkolben, ausgestattet mit Flügelrührer, Innenthermometer, Kühler und Tropftrichter mit Druckausgleich vorgelegt. Eine Lösung von 236,6 g (1,88 mol) Oxalsäuredihydrat in 2632 g vollentsalztem Wasser wurde bei 25 - 30°C und Normaldruck unter Rühren über 3 Stunden zugetropft. Während des Zutropfens bildete sich eine geringe Menge Schaum. Die freiwerdenden Gase wurden über den Kühler abgeleitet. Nach dem Zutropfen der Oxalsäurelösung wurde noch 2 Stunden bei 25°C gerührt. Auswaage: 4688 g.

Ein Teil (1000 g) der hellgelben, klaren Lösung wurde bei Normaldruck und bei Temperaturen zwischen etwa 80 bis 90°C zur Abdestillation von Methanol, Dimethylcarbonat und einem kleinen Teil des Wassers in einen Dünnschichtverdampfer getropft. Das abgelaufene Sumpfprodukt wurde mit etwas vollentsalztem Wasser versetzt, um den Solvensverlust auszugleichen. Das Sumpfprodukt wurde bei Temperaturen von 95 bis 105°C erneut wie oben beschrieben in einen Dünnschichtverdampfer getropft, um die Restmengen au Methanol und Dimethylcarbonat abzudestillieren. Falls erforderlich, kann die Dünnschichtverdampfer-Destillation und Wasserzugabe nochmals wiederholt werden. Das Endprodukt war eine 30 %ige Lösung von Tetradecyltrimethylammoniumoxalat in Wasser frei von Halogenidanionen.

### Beispiel 2:

123 g (0,50 mol) Tetradecyldimethylamin und 150 g (1,67 mol) Dimethylcarbonat wurden in einem 1 Liter-Glasrührautoklaven nach der Dichtigkeitsprüfung unter einer N₂-Schutzgasatmosphäre 8 Stunden bei 130°C und einem Eigendruck von 3 - 4 bar (absolut) gerührt. Bei Temperaturen über 110°C blieb der Autoklaveninhalt flüssig, unter 110°C wurde er langsam fest. Der Autoklaveninhalt wurde in einen 2 Liter-Glasrundkolben, ausgestattet mit Flügelrührer, Innenthermometer, Kühler und Tropftrichter mit Druckausgleich, eingefüllt und in Methanol gelöst. Eine konzentrierte Lösung von 63 g (0,50 mol) Oxalsäuredihydrat in destilliertem Wasser wurde bei Raumtemperatur unter langsamen Rühren so zugetropft, dass die Schaummenge nicht zu stark anstieg. Die freigesetzten Gase wurden über den Kühler abgeleitet. Nach dem Zutropfen der Oxalsäurelösung wurde der Kolbeninhalt noch eine halbe Stunde bei 50°C gerührt.

Die klare, blassgelbe Lösung wurde unter Rühren mit einem wandgängigen Rührer bei 60 bis 70°C und einem Druck von anfangs 900 mbar, der langsam auf etwa 100 mbar abgesenkt wurde, andestilliert. Ein zu starkes Konzentrieren und ein Überhitzen der Kolbenwand müssen vermieden werden, da dies zu unerwünschter Gelbildung führen kann. Nach dem Andestillieren wurde in quantitativer Ausbeute eine 35 %ige Lösung von Tetradecyltrimethylammoniumhydrogenoxalat in Wasser erhalten.

### Beispiel 3:

230 g (1,0 mol) Kokosalkyldimethylamin (Kokosalkyl: C₈H₁₇ bis C₁₈H₃₇), 126 g (1,4 mol) Dimethylcarbonat und 400 g Isopropanol wurden in einem 2 Liter-Edelstahl-Rührautoklaven nach der Dichtigskeitsprüfung unter einer N₂-Schutzgasatmosphäre 8 Stunden bei 140°C unter Eigendruck gerührt. Nach Abkühlen und Entspannen wurde der Autoklaveninhalt in einen 4 Liter-Glasrundkolben, ausgestattet mit Flügelrührer, Innenthermometer, Kühler und Tropftrichter mit Druckausgleich, eingefüllt. Eine Lösung von 64 g (0,51 mol) Oxalsäuredihydrat in VE-Wasser wurde bei 40°C unter Rühren langsam zugetropft und die freigesetzten Gase über den Kühler abgeleitet. Die klare, hellgelbe Lösung wurde bei 60°C und ca. 200 mbar Druck in einem Dünnschichtverdampfer aufkonzentriert. Nach zwei Durchgängen wurde als Dünnschichtverdampferablauf eine 41 %ige Lösung von Kokosalkyltrimethylammoniumoxalat in Wasser und Isopropanol erhalten.

## Patentansprüche

1. Verfahren zur Herstellung von quaternären Ammoniumoxalaten oder von quaternären Ammoniumhydrogenoxalaten der Formel (1) 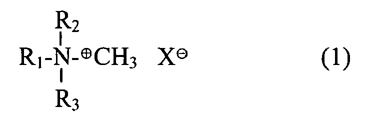 durch Umsetzung von Aminen der Formel (2) 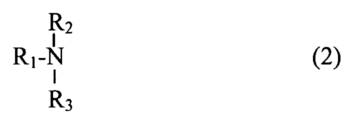 mit Dimethylcarbonat
und weitere Umsetzung des Zwischenprodukts mit Oxalsäure oder Oxalsäuredihydrat worin
X^{⊖} ½ C₂O₄^{2⊖} oder HC₂O₄^{⊖},
R₁ einen geradkettigen oder verzweigten, aliphatischen Kohlenwasserstoffrest mit 8 bis 22 C-Atomen, der gesättigt ist oder eine, zwei oder drei C=C-Doppelbindungen enthalten kann,
R₂ CH₃, C₂H₅, C₃H₇, C₄H₉ oder die für R₁ angegebene Bedeutung, und
R₃ CH₃, C₂H₅, C₃H₇, C₄H₉
bedeuten, wobei die Umsetzung des Amins der Formel (2) mit Dimethylcarbonat unter dem sich dabei im geschlossenen Reaktionsgefäß einstellenden Druck und die weitere Umsetzung des isolierten oder nicht isolierten Zwischenprodukts mit Oxalsäure oder Oxalsäuredihydrat in einem Solvens durchgeführt wird.

2. Verfahren gemäß Anspruch 1, bei dem die Umsetzung von Amin der Formel (2) mit Dimethylcarbonat in einem Alkohol, vorzugsweise in Methanol, als Solvens und in einem Molverhältnis von Amin der Formel (2) zu Dimethylcarbonat von 1:1 bis 1:4 oder in überschüssigem Dimethylcarbonat ohne Zusatz eines weiteren Solvens durchgeführt wird.

3. Verfahren gemäß Anspruch 1 und/oder 2, bei dem die Umsetzung von Amin der Formel (2) mit Dimethylcarbonat bei 100 bis 180°C unter dem autogenen Druck der Reaktionsmischung durchgeführt wird.

4. Verfahren gemäß einem oder mehreren der Ansprüche 1 bis 3, bei dem die weitere Umsetzung des isolierten oder nicht isolierten Zwischenprodukts mit Oxalsäure oder Oxalsäuredihydrat in einem Solvens ausgewählt aus der Gruppe Wasser, Methanol, Alkohol oder deren Mischungen bei 10 bis 80°C durchgeführt wird.

5. Verfahren gemäß einem oder mehreren der Ansprüche 1 bis 4, bei dem Oxalsäure oder Oxalsäuredihydrat als wässrige Lösung zum isolierten oder zum nicht isolierten Zwischenprodukt, erhalten aus der Umsetzung von Amin der Formel (2) mit Dimethylcarbonat so langsam und kontinuierlich dosiert wird, dass kein Schaum gebildet wird.

6. Verfahren gemäß einem oder mehreren der Ansprüche 1 bis 5, bei dem das Molverhältnis von Amin der Formel (2) zu Oxalsäure oder Oxalsäuredihydrat zwischen 0,9:1 und 2,1:1 liegt.

7. Verfahren gemäß einem oder mehreren der Ansprüche 1 bis 6, bei dem das Molverhältnis von Amin der Formel (2) zu Oxalsäure oder Oxalsäuredihydrat zur Herstellung eines quaternären Ammoniumoxalats zwischen 1,8:1 und 2,1:1 und zur Herstellung eines quaternären Ammoniumhydrogenoxalats zwischen 0,9:1 und 1,1:1 liegt.

8. Verfahren gemäß einem oder mehreren der Ansprüche 1 bis 7, bei dem das Amin der Formel (2) ein Alkyldimethylamin ist, wobei Alkyl CₙH₂ₙ₊₁ mit n = 8 bis 22 bedeutet oder ein Dialkylmethylamin ist, wobei Alkyl CₙH₂ₙ₊₁ mit n = 8 bis 14 bedeutet, und die Alkylgruppen gleich oder verschieden sein können.

9. Verfahren gemäß einem oder mehreren der Ansprüche 1 bis 8, bei dem das Amin der Formel (2) ein Tetradecyldimethylamin C₁₄H₂₉N(CH₃)₂ ist.

10. Verfahren gemäß einem oder mehreren der Ansprüche 1 bis 9, bei dem nach der Umsetzung mit Oxalsäure oder Oxalsäuredihydrat ein oder mehrere Solventien, insbesondere Methanol, in einem einstufigen oder mehrstufigen Dünnschichtverdampfer abdestilliert werden.

## Claims

1. A method for the production of quaternary ammonium oxalates or of quaternary ammonium hydrogen oxalates of formula (1) 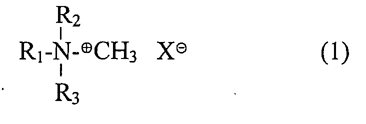 by reacting amines of formula (2) 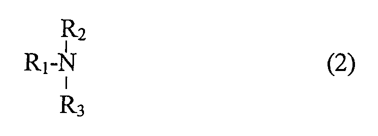 with dimethyl carbonate
and further reacting the intermediate product with oxalic acid or oxalic acid dihydrate, in which
X^{⊖} is ½ C₂O₄^{2⊖} or HC₂O₄^{⊖},
R₁ is a straight-chained or branched, aliphatic hydrocarbon radical having 8 to 22 carbon atoms, which is saturated or can contain one, two or three C=C-double bonds,
R₂ is CH₃, C₂H₅, C₃H₇, C₄H₉ or the meaning given for R₁, and
R₃ is CH₃, C₂H₅, C₃H₇, C₄H₉,
where the reaction of the amine of formula (2) with dimethyl carbonate is carried out under the pressure which is established here in the closed reaction vessel, and the further reaction of the isolated or nonisolated intermediate product with oxalic acid or oxalic acid dihydrate is carried out in a solvent.

2. The method as claimed in claim 1, in which the reaction of amine of formula (2) with dimethyl carbonate is carried out in an alcohol, preferably in methanol, as solvent and in a molar ratio of amine of formula (2) to dimethyl carbonate of from 1:1 to 1:4 or in excess dimethyl carbonate without the addition of a further solvent.

3. The method as claimed in claim 1 and/or 2, in which the reaction of amine of formula (2) with dimethyl carbonate is carried out at 100 to 180°C under the autogenous pressure of the reaction mixture.

4. The method as claimed in one or more of claims 1 to 3, in which the further reaction of the isolated or nonisolated intermediate product with oxalic acid or oxalic acid dihydrate is carried out in a solvent chosen from the group water, methanol, alcohol or mixtures thereof at 10 to 80°C.

5. The method as claimed in one or more of claims 1 to 4, in which oxalic acid or oxalic acid dihydrate is metered as aqueous solution into the isolated or nonisolated intermediate product obtained from the reaction of amine of formula (2) with dimethyl carbonate sufficiently slowly and continuously that no foam is formed.

6. The method as claimed in one or more of claims 1 to 5, in which the molar ratio of amine of formula (2) to oxalic acid or oxalic acid dihydrate is between 0.9:1 and 2.1:1.

7. The method as claimed in one or more of claims 1 to 6, in which the molar ratio of amine of formula (2) to oxalic acid or oxalic acid dihydrate for producing a quaternary ammonium oxalate is between 1.8:1 and 2.1:1 and for producing a quaternary ammonium hydrogen oxalate is between 0.9:1 and 1.1:1.

8. The method as claimed in one or more of claims 1 to 7, in which the amine of formula (2) is an alkyldimethylamine, where alkyl is CₙH₂ₙ₊₁ where n = 8 to 22 or is a dialkylmethylamine, where alkyl is CₙH₂ₙ₊₁ where n = 8 to 14, and the alkyl groups may be identical or different.

9. The method as claimed in one or more of claims 1 to 8, in which the amine of formula (2) is a tetradecyldimethylamine C₁₄H₂₉N(CH₃)₂.

10. The method as claimed in one or more of claims 1 to 9, in which, following the reaction with oxalic acid or oxalic acid dihydrate, one or more solvents, in particular methanol, are distilled off in a single-stage or multistage thin-film evaporator.

## Revendications

1. Procédé pour la préparation d'oxalates d'ammonium quaternaire ou d'hydrogénooxalates d'ammonium quaternaire de formule (1) 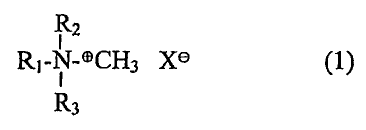 par transformation d'amines de formule (2) 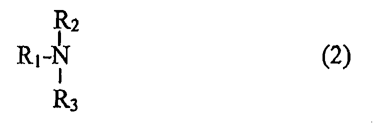 avec du carbonate de diméthyle et par transformation ultérieure du produit intermédiaire avec de l'acide oxalique ou de l'acide oxalique dihydraté, dans lesquelles
X^{⊖} 1/2 C₂O₄^{2⊖} ou HC₂O₄^{⊖},
R₁ signifie un radical hydrocarboné linéaire ou ramifié, aliphatique comprenant 8 à 22 atomes de carbone, qui est saturé ou qui peut contenir une, deux ou trois doubles liaisons C=C,
R₂ signifie CH₃, C₂H₅, C₃H₇, C₄H₉ ou la signification indiquée pour R¹ et
R₃ signifie CH₃, C₂H₅, C₃H₇, C₄H₉
la transformation de l'amine de formule (2) avec du carbonate de diméthyle étant réalisée à la pression qui se règle dans le récipient de réaction fermé et la transformation ultérieure du produit intermédiaire isolé ou non isolé étant réalisée avec de l'acide oxalique ou de l'acide oxalique dihydraté dans un solvant.

2. Procédé selon la revendication 1, dans lequel la transformation de l'amine de formule (2) avec du carbonate de diméthyle est réalisée dans un alcool, de préférence dans du méthanol, comme solvant et à un rapport molaire de l'amine de formule (2) au carbonate de diméthyle de 1:1 à 1:4 ou dans du carbonate de diméthyle en excès sans addition d'un autre solvant.

3. Procédé selon la revendication 1 et/ou 2, dans lequel la transformation de l'amine de formule (2) avec du carbonate de diméthyle est réalisée à 100 jusqu'à 180°C sous la pression autogène du mélange réactionnel.

4. Procédé selon l'une ou plusieurs des revendications 1 à 3, dans lequel la transformation ultérieure du produit intermédiaire isolé ou non isolé avec de l'acide oxalique ou de l'acide oxalique dihydraté est réalisée dans un solvant choisi dans le groupe formé par l'eau, le méthanol, un alcool ou leurs mélanges à 10 jusqu'à 80°C.

5. Procédé selon l'une ou plusieurs des revendications 1 à 4, dans lequel l'acide oxalique ou l'acide oxalique dihydraté est ajouté sous forme de solution aqueuse au produit intermédiaire isolé ou non isolé, obtenu à partir de la transformation de l'amine de formule (2) avec du carbonate de diméthyle, lentement et en continu de manière telle qu'il ne se forme pas de mousse.

6. Procédé selon l'une ou plusieurs des revendications 1 à 5, dans lequel le rapport molaire de l'amine de formule (2) à l'acide oxalique ou à l'acide oxalique dihydraté est situé entre 0,9:1 et 2,1:1.

7. Procédé selon l'une ou plusieurs des revendications 1 à 6, dans lequel le rapport molaire de l'amine de formule (2) à l'acide oxalique ou à l'acide oxalique dihydraté, pour la préparation d'un oxalate d'ammonium quaternaire, est situé entre 1,8:1 et 2,1:1 et, pour la préparation d'un hydrogénooxalate d'ammonium quaternaire, entre 0, 9:1 et 1,1:1.

8. Procédé selon l'une ou plusieurs des revendications 1 à 7, dans lequel l'amine de formule (2) est une alkyldiméthylamine, alkyle signifiant CₙH₂ₙ₊₁ avec n = 8 à 22 ou une dialkylméthylamine, alkyle signifiant CₙH₂ₙ₊₁ avec n = 8 à 14, et les groupes alkyle pouvant être identiques ou différents.

9. Procédé selon l'une ou plusieurs des revendications 1 à 8, dans lequel l'amine de formule (2) est une tétradécyldiméthylamine C₁₄H₂₉N(CH₃)₂.

10. Procédé selon l'une ou plusieurs des revendications 1 à 9, dans lequel, après la transformation avec de l'acide oxalique ou de l'acide oxalique dihydraté, un ou plusieurs solvants, en particulier le méthanol, sont éliminés par distillation dans un évaporateur à couche mince à un ou plusieurs étages.
